# EUROPEAN PATENT SPECIFICATION

(11) **EP 1 748 312 B1**
(45) Date of publication and mention of the grant of the patent: **10.09.2008**
(21) Application number: 06015595.9
(22) Date of filing: 26.07.2006
(51) Int. Cl.: G02F 1/355, A61F 9/008, A61N 5/067, G02F 1/37, H01S 3/067, G02F 1/35

(54) **Medical laser apparatus**
Medizinischer Laser Vorrichtung
Appareil laser médical

(30) Priority: 29.07.2005 JP 2005220697
(43) Date of publication of application: 31.01.2007
(73) Proprietor: NIDEK CO., LTD, Hiroishi-cho, Gamagori-shi Aichi-ken 443-0038 (JP)
(72) Inventor: Hayashi, Kenichi, Gamagori-shi, Aichi-ken 443-0038 (JP); Yamada, Tsuyoshi, Gamagori-shi, Aichi-ken 443-0038 (JP)
(74) Representative: Materne, Jürgen

(56) References cited:
- EP-A- 1 281 378
- EP-A- 1 471 613
- WO-A-92/04871
- FR-A- 2 864 903
- US-A- 3 962 576
- US-A- 5 260 953
- US-A1- 2002 111 611
- US-B1- 6 614 584
- KLINER D A V ET AL: "Efficient second, third, fourth, and fifth harmonic generation of a Yb-doped fiber amplifier" OPTICS COMMUNICATIONS, NORTH-HOLLAND PUBLISHING CO. AMSTERDAM, NL, vol. 210, no. 3-6, 15 September 2002 (2002-09-15), pages 393-398, XP004380478 ISSN: 0030-4018

## Description

### BACKGROUND OF THE INVENTION

### 1. Field of the Invention

The present invention relates to a medical laser apparatus for use in an ophthalmic field and others.

### 2. Description of Related Art

In the ophthalmic field, there is a laser apparatus adapted to wavelength-convert an infrared fundamental laser beam emitted from a laser source to a visible second harmonic laser beam by a wavelength converting element, and perform treatment with the visible beam (or a visible or ultraviolet laser beam generated by additional wavelength-conversion).

Such laser apparatus has some problems if a fiber laser source is used as the laser source. Specifically, a central wavelength of the fundamental beam tends to shift due to a change in power thereof, resulting in a deterioration in wavelength-conversion efficiency of the wavelength converting element in converting the fundamental beam to the second harmonic beam.

In particular, the apparatus using a Raman fiber laser source by stimulated Raman scattering effect would cause a large shift of a central wavelength of a fundamental beam due to a change in power thereof.

Document US 3,962,576 discloses an harmonic generator crystal for a tunable laser which measures the primary (fundamental) and secondary (second harmonic) radiation on photoelectric detectors to adjust the angle of incidence of the fundamental upon the crystal and corrects for the displacement of the beam arising from the tilting of the doubling crystal.

Document FR2864903 discloses a variable power medical fiber laser system.

Document EP1471613 and EP1281378 disclose a medical fiber laser system whose fundamental output is frequency doubled and whose output power controlled by the operator, but do not posses optical axis deviation correcting means with control means for controlling the correcting means based on a change in power of the fundamental.

Document US 5,260,953 discloses a YAG-pumped cavity for frequency doubling the infrared fundamental in which the angle of incidence of the doubling crystal is changed to ensure resonance of a certain fundamental, as detected by an array, and in which a counter-rotating identical second crystal is used to correct any deviation in the optical paths arising from the first doubling crystal.

### BRIEF SUMMARY OF THE INVENTION

The present invention has been made in view of the above circumstances and has an object to provide a medical laser apparatus capable of efficiently and stably performing wavelength-conversion of a fundamental laser beam to a second harmonic laser beam even when a central wavelength of the fundamental laser beam shifts due to a change in power thereof.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the purpose of the invention, there is provided a medical laser apparatus for performing treatment by irradiating an affected part with a laser beam, the apparatus comprising: a fiber laser source with variable output power, which emits an infrared fundamental laser beam; a wavelength converting element which wavelength-converts the fundamental beam to a visible second harmonic laser beam and is placed on an optical axis of the fundamental beam; first incident angle changing means for changing an incident angle of the fundamental beam to the wavelength converting element; optical axis deviation correcting means for correcting deviation of an optical axis of the second harmonic beam caused by a change of the incident angle of the fundamental beam to the wavelength converting element; and control means for controlling the first changing means and the correcting means based on a change in power of the fundamental beam.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1 is a schematic structural view of an ophthalmic laser photocoagulation apparatus in the present embodiment;
Fig. 2 is an explanatory view showing an adjustment method, using two wavelength converting elements, for a shift of a central wavelength of a fundamental laser beam caused by a change in power thereof; and
Fig. 3 is an explanatory view showing an adjustment method, using a single wavelength converting element and a single optical member, for a shift of a central wavelength of a fundamental laser beam caused by a change in power thereof.

### DETAILED DESCRIPTION OF THE PREFERRED EMBODIMENTS

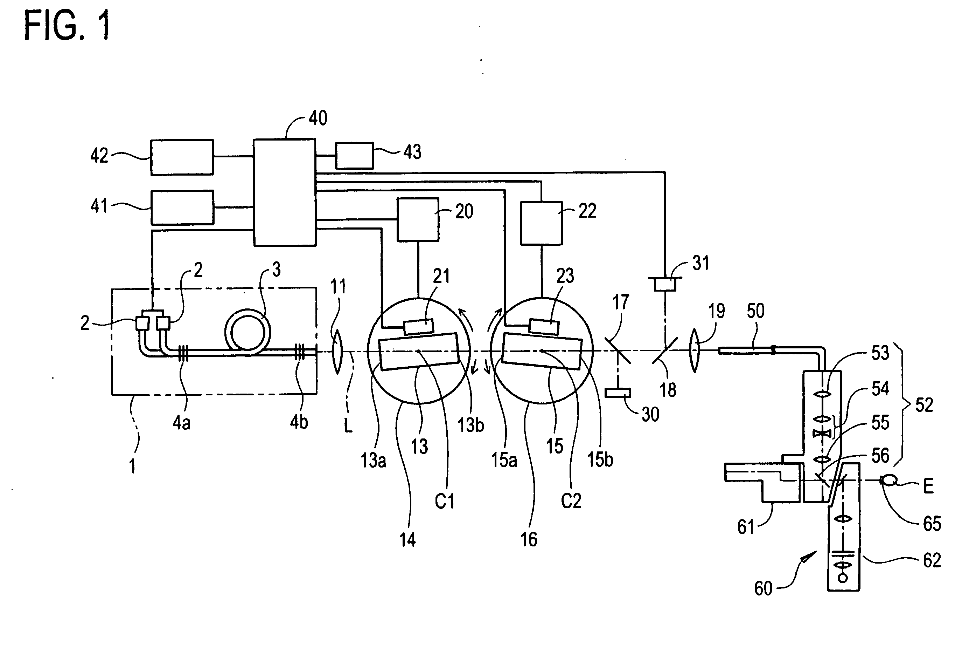

A detailed description of a preferred embodiment of the present invention will now be given referring to the accompanying drawings. Fig. 1 is a schematic structural view of an ophthalmic laser photocoagulation apparatus in the present embodiment. The present invention may be applied to not only the ophthalmic laser photocoagulation apparatus but another laser apparatus for ophthalmic treatment or a laser apparatus for use in another medical field.

A fiber laser source 1 which emits an infrared fundamental laser beam includes a plurality of laser diodes (LD) 2 for excitation and a Yb-doped fiber 3 for a resonator. The fiber 3 is formed with for example two fiber Bragg gratings (FBG) 4a and 4b forming the resonator to emit the fundamental beam having a central wavelength λ1 of 1160 nm by excitation light having a wavelength of 890 nm from the LD 2. The power of the fundamental beam to be emitted from the laser source 1 is adjusted when a controller 40 serving as control means controls the power of the excitation light from the LD 2.

On an optical path of the fundamental beam emitted from the laser source 1, there are arranged a collimator lens 11 and a first wavelength converting element 13. The fundamental beam is made into a parallel or converged beam by the lens 11 to enter the first wavelength converting element 13 in which the beam is wavelength-converted to a visible second harmonic laser beam having a central wavelength λ2 of 580 nm.

On an optical path of the second harmonic beam emerging from the first wavelength converting element 13, a second wavelength converting element 15 is arranged. The second harmonic beam enters the second wavelength converting element 15 and passes therethrough without conversion. Further, a remaining fundamental beam having not been wavelength-converted by the first wavelength converting element 13 enters the second wavelength converting element 15 in which the beam is wavelength-converted to the second harmonic beam.

On an optical path of the second harmonic beam emerging from the second wavelength converting element 15, there are arranged a dichroic mirror 17 serving as a beam splitter and having a property of reflecting infrared light and transmitting visible light, a half mirror 18 serving as a beam splitter and having a property of reflecting a part of visible light but transmitting most part of the visible light, a condensing lens 19, and an optical fiber 50. A damper 30 for the fundamental beam is placed in a reflection direction by the mirror 17. Further, a sensor 31 for detecting the power of the second harmonic beam is placed in a reflection direction by the mirror 18. The second harmonic beam passes through the mirror 17 and then most part of the beam passes through the mirror 18 to enter the fiber 50 via the lens 19. A part of the second harmonic beam reflected by the mirror 18 enters the sensor 31. A remaining fundamental beam having still not been wavelength-converted by the second wavelength converting element 15 is reflected by the mirror 17 to enter the damper 30.

The second harmonic beam having entered the fiber 50 is then irradiated to a fundus of a patient's eye E through a light delivery optical system 52 and a contact lens 65. The light delivery optical system 52 includes a relay lens 53, a zoom lens system 54 for changing the spot size of the beam, an objective lens 55, and a mirror 56 which reflects the beam to the eye E. This light delivery optical system 52 is attached to a slit lamp 60 having a binocular microscope part 61 and an illumination part 62.

Used as the first wavelength converting element 13 is a quasi phase matching element (hereinafter, referred to as "QPM element") having a periodic polarization inverting structure. As a material of this QPM, LiNb0₃, LiTa0₃, KTiOP0₄, or a crystal is preferably used. The QPM element serving as the first wavelength converting element 13 is structured such that a polarization direction of a nonlinear optical material is inverted alternately at every coherence length to satisfy quasi phase matching. The periodic structure is determined so as to efficiently wavelength-convert a fundamental beam to a second harmonic beam. The QPM element as the first wavelength converting element 13 includes a beam entrance face 13a and a beam exit face 13b parallel to each other.

As the second wavelength converting element 15, an identical QPM element to the first wavelength converting element 13 is used. In this case, however, it is preferable to perform phase correction between the first and second wavelength converting elements 13 and 15. For the second wavelength converting element 15 of a bulk type, no phase correction is required. Thus, an apparatus structure can be simplified. The QPM element serving as the second wavelength converting element 15 similarly includes a beam entrance face 15a and a beam exit face 15b parallel to each other.

The first wavelength converting element 13 is mounted on a rotating base 14 whose rotation center C1 is on a reference optical axis L of the beam. This rotation center C1 is located at the center of the first wavelength converting element 13. The rotating base 14 is rotated by a rotation driving part 20 including a motor, a gear, and others. Simultaneously the first wavelength converting element 13 is also rotated about the rotation center C1, changing the angles of the entrance face 13a and the exit face 13b with respect to the optical axis L. Similarly, the second wavelength converting element 15 is mounted on a rotating base 16 whose rotation center C2 is on the optical axis L. This rotation center C2 is located at the center of the second wavelength converting element 15. The rotating base 16 is rotated by a rotation driving part 22 including a motor, a gear, and others. Simultaneously the second wavelength converting element 15 is also rotated about the rotation center C2, changing the angles of the entrance face 15a and the exit face 15b with respect to the optical axis L. Note that the reference optical axis L corresponds to an optical axis of the fundamental beam emitted from the laser source 1 in the case where the wavelength converting elements 13 and 15 are absent and also to an optical axis of the wavelength-converted second harmonic beam in the case where the wavelength converting elements 13 and 15 are arranged colinear (where each entrance face and each exit face are perpendicular to the optical axis).

Furthermore, a temperature controller 21 for regulating (changing) the temperature of the first wavelength converting element 13 is mounted on the rotating base 14. A temperature controller 22 for regulating (changing) the temperature of the second wavelength converting element 15 is mounted on the rotating base 16. Each of the temperature controllers 21 and 23 is constituted of e.g. a Peltier device. The wavelength converting elements 13 and 15 are maintained at a predetermined constant temperature by the temperature controllers 21 and 23 respectively so that their periodic structures will not vary with temperature changes.

The laser source 1, the rotation driving parts 20 and 22, the temperature controllers 21 and 23 are connected to the controller 40 which controls them individually. Further connected to the controller 40 are an input part 41 for inputting beam irradiation conditions and others such as the power of the second harmonic beam to be used as a treatment beam, a trigger switch 42 to start beam irradiation, and a memory 43.

The following explanation will be made on an adjustment method for a shift of the central wavelength of the fundamental beam caused by a change in power thereof.

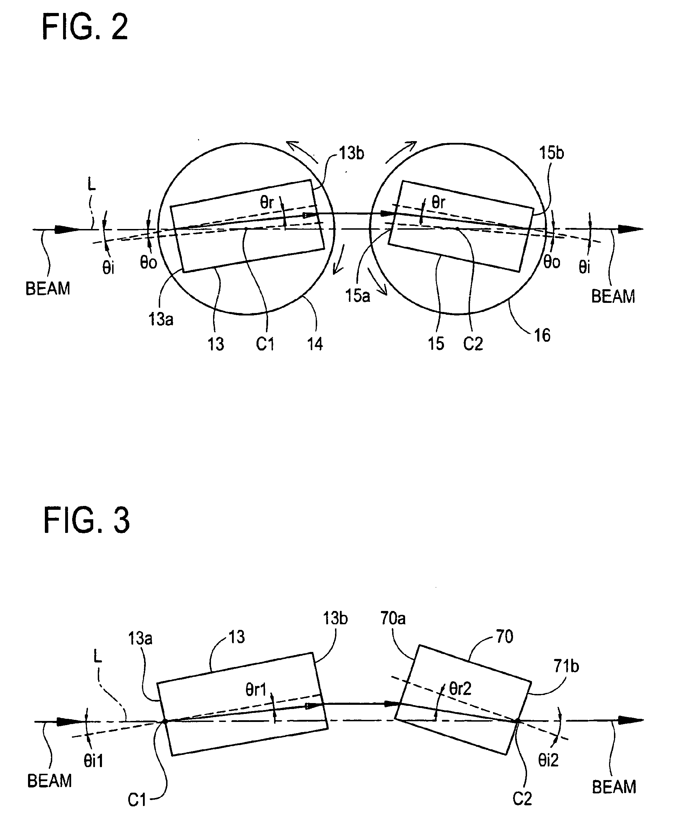

For adjustment for the case where the central wavelength λ1 of the fundamental beam shifts to a long wavelength side or a short wavelength side, a reference position of the first wavelength converting element 13 is a position inclined at a predetermined angle θo (e.g. 5°) to the optical axis L as shown in Fig. 2. The periodic structure of the first wavelength converting element 13 is determined so that the fundamental beam is wavelength-converted to the second harmonic beam when passes through the first wavelength converting element 13 in this position. This determination is also made by incorporating a variation in optical path length by refraction, as will be mentioned later. Similarly, a reference position of the second wavelength converting element 15 is a position inclined at the angle θo in a direction opposite to the first wavelength converting element 13 with respect to the optical axis L.

The case where the central wavelength λ1 of the fundamental beam shifts to the long wavelength side is first explained below.

When the rotating base 14 is rotated at a rotation angle θi with respect to the optical axis L to satisfy a relation; θi > θo, an incident (entrance) angle of the fundamental beam with respect to the entrance face 13a of the first wavelength converting element 13 (i.e. an angle with respect to the normal to the entrance face 13a) is inclined to the angle θi. The fundamental beam having entered the first wavelength converting element 13 in this position travels longer through the inside of the first wavelength converting element 13 than the case where the first wavelength converting element 13 is inclined at the angle θo. Specifically, an optical path for wavelength-conversion corresponding to an equivalently long wavelength is formed in the first wavelength converting element 13. By controlling the angle θi to provide the optical path length corresponding to the shift amount of the central wavelength λ1 of the fundamental beam, it is possible to prevent a deterioration in wavelength-conversion efficiency. An angle θr (an angle with respect to the normal to the entrance face 13a) of the beam passing through the inside of the first wavelength converting element 13 inclined at the angle θi can be calculated based on a known refractive index of the first wavelength converting element 13. Based on this angle θr, the optical path length for wavelength-conversion in the inside of the first wavelength converting element 13 can also be calculated.

When the first wavelength converting element 13 is inclined at the angle θi, the optical axis of the second harmonic beam (and the remaining fundamental beam) emerging from the exit face 13b will deviate from the optical axis L. To correct this deviation, accordingly, the rotating base 16 is rotated at the angle θi with respect to the optical axis L in the opposite direction to the rotation angle θi of the rotating base 14, thereby inclining an incident (entrance) angle (an angle with respect to the normal to the entrance face 15a) of the second harmonic beam (and the remaining fundamental beam) emerging from the exit face 13b of the first wavelength converting element 13 to an angle θi in the same opposite direction as above with respect to the entrance face 15a of the second wavelength converting element 15. Thus, the second harmonic beam (and the remaining fundamental beam) entering the entrance face 15a of the second wavelength converting element 15 is deflected at the angle θr. Since the first and second wavelength converting elements 13 and 15 are equal in length, the optical axis of the second harmonic beam (and the remaining fundamental beam) emerging from the exit face 15b of the second wavelength converting element 15 is returned to the optical axis L.

When the optical axis of the second harmonic beam emerging from the second wavelength converting element 15 is returned to the optical axis L, the second harmonic beam is made by the lens 19 to enter the fiber 50 while the beam is in the same condition as before the optical path length is changed. This makes it possible to solve disadvantages resulting from entrance deviation. In other words, if the beam as deviated enters the fiber 50, a beam transmission efficiency of the fiber 50 will become down. Further, this may cause inhomogeneous beam intensity distribution, resulting in difficulty in forming an uniform coagulation spot.

Next, the case where the central wavelength λ1 of the fundamental beam shifts to the short wavelength side is explained below.

In this case, contrary to the above mentioned case, the rotating base 14 is rotated at the rotation angle θi with respect to the optical axis L to satisfy a relation; θi < θo. Accordingly, the optical path length of the fundamental beam which travels through the inside of the first wavelength converting element 13 is shorter than the case where the first converting element 13 is inclined at the angle θo. The rotating base 16 is thus rotated at the angle θi in the opposite direction to the rotation angle θi of the rotating base 14. This makes it possible to correct the deviation of the optical axis caused by inclination of the first wavelength converting element 13.

Inclining each of the wavelength converting elements 13 and 15 as mentioned above makes it possible to adjust the problem that the central wavelength λ1 of the fundamental beam shifts. As another adjustment method for this problem, a method of controlling the temperature of the first wavelength converting element 13 is also conceivable. However, in the temperature control, it is necessary to largely change the temperature as the power of the fundamental beam is largely changed. It therefore problematically takes long before the changed temperature becomes stable at a constant temperature. On the other hand, the method that the wavelength converting elements 13 and 15 are inclined as described above can rapidly adjust such problem by electrical driving of a motor or the like.

In the above embodiment, the wavelength converting elements 13 and 15 are maintained at the predetermined temperatures by the temperature controllers 21 and 23 respectively. Further, addition of a temperature control function is more convenient. For instance, in order to adjust a fine wavelength-shift over a long term resulting from drifts, the temperature controllers 21 and 23 may be adapted to precisely control the temperature to be maintained constant so that the power of the second harmonic beam to be detected by the sensor 31 is maximum.

Operations of the apparatus during treatment will be explained below. For treatment, the beam irradiation conditions are set with the input part 41. In the present embodiment, the power of the second harmonic beam serving as the treatment beam can be set in a range of 50 mmW to 1.4 W. Assuming that the wavelength-conversion efficiency of each of the wavelength converting elements 13 and 15 is 20%, the power of the fundamental beam from the laser source 1 is adjusted in a range of 250 mmW to 7W. Stored in the memory 43 is a relationship between a change in power of the fundamental beam from the laser source 1 (a change in power of the excitation light, i.e. a change in driving current of the LD 2), which have been obtained in advance by experiment or the like, and the rotation angle θi of each of the rotating bases 14 and 16 to incline the associated wavelength converting elements 13 and 15. When the power of the treatment beam (the second harmonic beam) is set, the controller 40 calculates the corresponding driving current of the LD 2 and reads out the angle θi corresponding to the power of the fundamental beam to be emitted from the laser source 1 by driving of the LD 2, and controls the driving of the rotation driving parts 20 and 22 to rotate the rotating bases 14 and 16 respectively, thereby inclining the associated wavelength converting elements 13 and 15.

When a command signal for beam irradiation is inputted with a switch on the input part 41, the controller 40 drives the LD 2 to emit the fundamental beam from the laser source 1 and open a shutter not shown placed between the mirror 18 and the lens 19 (or move the shutter out of the optical path). Thus, the second harmonic beam is allowed to enter the fiber 50. The power of the second harmonic beam is detected by the sensor 31. The controller 40 fine adjusts the driving of the LD 2 to make the power stable at the set power. Further, it is preferable to further control the driving of the rotation driving parts 20 and 22 in accordance with the fine adjustment of the LD 2 to adjust the inclination of the wavelength converting elements 13 and 15 respectively.

The present invention may be embodied in other specific forms without departing from the spirit or essential characteristics thereof.

For instance, in the above explanation, the rotation center C1 of the rotating base 14 is centrally located in the first wavelength converting element 13 and the rotation center C2 of the rotating base 16 is centrally located in the second wavelength converting element 15. However, if the rotation angle θi is too large, the entrance face 13a of the first wavelength converting element 13 and the exit face 15b of the second wavelength converting element 15 will deviate from the optical axis L. To avoid this, the rotation center C1 may be located on the entrance face 13a and the rotation center C2 may be located on the exit face 15b.

As the means for correcting deviation of the optical axis of the second harmonic beam, an optical member having a refractive index and length equal to those of the first wavelength converting element 13 and having parallel faces serving as a beam entrance face and a beam exit face (i.e. an optical member enabling at least the second harmonic beam to pass therethrough) may be used instead of the second wavelength converting element 15 identical to the first wavelength converting element 13. In this case, the control of inclination of the optical member may be performed in a similar way to the control over the second wavelength converting element 15.

In the case where an optical member different in refractive index from the first wavelength converting element 13 is used instead of the second wavelength converting element 15, the difference in refractive index may be adjusted based on at least one of the length (a distance between the parallel entrance and exit faces) and the inclination of the optical member. Fig. 3 shows an example that, as the means for correcting the optical axis deviation, an optical member (an optical element) 70 different in refractive index from the first wavelength converting element 13 is used instead of the second wavelength converting element 15. In this example, the rotation center C1 is located on the entrance face 13a of the first wavelength converting element 13 and the rotation center C2 is located on a beam exit face 70b of the optical element 70. In this figure, the rotating bases and their rotation driving parts are not shown.

The optical member 70 is inclined at an angle θi2 to return the deviated optical axis of the second harmonic beam emerging from the exit face 13b of the first wavelength converting element 13 inclined at an angle θi1 to the optical axis L. This angle θi2 is determined based on a relationship between a refractive index of the optical member 70 to the second harmonic beam and the length of the optical member 70 (a distance between a beam entrance face 70a and the exit face 70b). The angle θi2 with respect to the angle θi1 has been calculated in advance and stored in the memory 43.

In the above explanation, the optical axis of the lens 19 whereby the second harmonic beam is allowed to enter the fiber 50 is located to be coaxial with the optical axis L (the optical axis of the lens 11). However, both optical axes do not always have to be coaxial. In the case where the optical axis of the lens 19 has been deviated originally from the optical axis L, the inclination of the correcting member for optical axis deviation such as the second wavelength converting element 15 and the optical member 70 has only to be adjusted to conform with the optical axis of the lens 19.

In the above explanation, the first and second wavelength converting elements 13 and 15 and the optical member 70 are configured to have a beam entrance face and a beam exit face parallel to each other. This is because when the entrance face and the exit face are parallel to each other, an outgoing beam from the exit face shifts in parallel from an incoming beam on the entrance face, so that the inclination angle for correcting the optical axis deviation can be calculated easily; however, another configuration may also be adopted. Even when the entrance face and the exit face are not parallel to each other, if a relation between them is well known, the optical axis direction of the outgoing beam from the exit face can be calculated based on that relation and the inclination angle of the optical axis deviation correcting member such as the second wavelength converting element 15 and the optical member 70 can be determined to correct the optical axis deviation.

In the above explanation, the first and second wavelength converting elements 13 and 15 and the optical member 70 are inclined by rotation, but they may be swung in a range that at least the beam may enter it.

While the presently preferred embodiment of the present invention has been shown and described, it is to be understood that this disclosure is for the purpose of illustration and that various changes and modifications may be made without departing from the scope of the invention as set forth in the appended claims.

## Claims

1. A medical laser apparatus for performing treatment by irradiating an affected part with a laser beam, the apparatus comprising:
a fiber laser source (1) with variable output power, which emits an infrared fundamental laser beam;
a wavelength converting element (13) which wavelength-converts the fundamental beam to a visible second harmonic laser beam and is placed on an optical axis of the fundamental beam;
first incident angle changing means (14, 20) for changing an incident angle of the fundamental beam to the wavelength converting element (13);
optical axis deviation correcting means (15, 16, 22, 70) for correcting deviation of an optical axis of the second harmonic beam caused by a change of the incident angle of the fundamental beam to the wavelength converting element (13); and
control means (40) for controlling the first changing means (14, 20) and the correcting means (15, 16, 22, 70) based on a change in power of the fundamental beam.

2. The medical laser apparatus according to claim 1, wherein the correcting means (15, 16, 22, 70) includes an optical member (15, 70) that has a predetermined refractive index and length and is placed on an optical path of the second harmonic beam and second incident angle changing means (16, 22) for changing an incident angle of the second harmonic beam to the optical member (15, 70), and
the control means (40) controls the first changing means (14, 20) and the second changing means (16, 22) based on the change in power of the fundamental beam.

3. The medical laser apparatus according to claim 2, wherein
the optical member (15) is equal in refractive index and length to the wavelength converting element (13), and
the control means (40) controls the first changing means (14, 20) and the second changing means (16, 22) to change the incident angle of the fundamental beam to the wavelength converting element and the incident angle of the second harmonic beam to the optical member so that both angles are equal, but opposite in direction.

4. The medical laser apparatus according to any of claims 1 to 3, further comprising:
temperature control means (21, 23) for changing a temperature of the wavelength converting element (13); and
a power sensor (31) which detects power of the second harmonic beam; and
wherein the control means controls the temperature control means (21, 23) based on a detection result of the power sensor.

## Patentansprüche

1. Medizinisches Lasergerät zum Ausführung von Behandlung durch Bestrahlung eines befallenen Teiles mit einem Laserstrahl, wobei das Gerät aufweist:
eine Faserlaserquelle (1) mit variabler Ausgabeleistung, die einen infraroten Fundamentallaserstrahl emittiert;
ein Wandlungselement (13) einer Wellenlänge, das den Fundamentallaserstrahl in einen sichtbaren Laserstrahl der zweiten Harmonischen in Wellenlänge wandelt und auf einer optischen Achse des Fundamentalstrahles gesetzt ist;
ein erstes Änderungsmittel (14, 20) eines Einstellwinkels zum Ändern eines Einstellwinkels des Fundamentalstrahles zu dem Wandlungselement (13) der Wellenlänge;
ein Korrigiermittel (15, 16, 22, 70) einer Abweichung einer optischen Achse zum Korrigieren Abweichung einer optischen Achse des Strahles der zweiten Harmonischen, die durch Änderung des Einfallswinkels des Fundamentalstrahles zum Wandlungselement (13) der Wellenlänge verursacht ist; und
ein Steuermittel (40) zum Steuern des ersten Änderungsmittels (14, 20) und des Korrigiermittels (15, 16, 22, 70) auf der Grundlage einer Änderung in Leistung des Fundamentalstrahles.

2. Medizinisches Lasergerät nach Anspruch 1, bei dem das Korrigiermittel (15, 16, 22, 70) ein optisches Teil (15, 70), das einen vorbestimmten Brechungsindex und Länge aufweist und auf dem optischen Pfad des Strahles der zweiten Harmonischen gesetzt ist, und ein zweites Änderungsmittel (16, 22) eines Einfallswinkels zum Ändern eines Einfallswinkels des Strahles der zweiten Harmonischen zu dem optischen Teil (15, 70), enthält, und
das Steuermittel (40) das erste Änderungsmittel (14, 20) und das zweite Änderungsmittel (16, 22) auf der Grundlage der Änderung in Leistung des Fundamentalstrahles steuert.

3. Medizinisches Lasergerät nach Anspruch 2, bei dem das optische Teil (15) in Brechungsindex und Länge gleich zu dem Wandlungselement (13) der Wellenlänge ist, und
das Steuermittel (40) das erste Änderungsmittel (14, 20) und das zweite Änderungsmittel (16, 22) steuert zum Ändern des Einfallswinkels des Fundamentalstrahles zu dem Wandlungselement der Wellenlänge und den Einfallswinkel des Strahles der zweiten Harmonischen zu dem optischen Teil so steuert, daß beide Winkel gleich sind aber entgegengesetzt in der Richtung sind.

4. Medizinisches Lasergerät nach einem der Ansprüche 1 bis 3, weiter mit:
einem Temperatursteuermittel (21, 23) zum Ändern einer Temperatur des Wandlungselementes (13) der Wellenlänge; und
einem Leistungssensor (31), der Leistung des Strahles der zweiten Harmonischen erfaßt; und
worin das Steuermittel das Temperatursteuermittel (21, 23) auf der Grundlage eines Erfassungsresultates des Leistungssensors steuert.

## Revendications

1. Appareil laser médical destiné à effectuer un traitement en irradiant une partie affectée avec un faisceau laser, l'appareil comprenant :
une source de laser à fibres (1) ayant une puissance de sortie variable, qui émet un faisceau laser fondamental infrarouge ;
un élément de conversion de longueur d'onde (13) qui convertit la longueur d'onde du faisceau fondamental en un faisceau laser de second harmonique visible et est placé sur un axe optique du faisceau fondamental ;
un premier moyen de changement d'angle d'incidence (14, 20) destiné à changer un angle d'incidence du faisceau fondamental par rapport à l'élément de conversion de longueur d'onde (13) ;
un moyen de correction de déviation de l'axe optique (15, 16, 22, 70) destiné à corriger la déviation d'un axe optique du faisceau de second harmonique provoquée par un changement de l'angle d'incidence du faisceau fondamental par rapport à l'élément de conversion de longueur d'onde (13) ; et
un moyen de commande (40) destiné à contrôler le premier moyen de changement (14, 20) et le moyen de correction (15, 16, 22, 70) sur la base d'un changement de puissance du faisceau fondamental.

2. Appareil laser médical selon la revendication 1, dans lequel le moyen de correction (15, 16, 22, 70) comprend un élément optique (15, 70) qui possède un indice de réfraction et une longueur prédéterminés et est placé sur un trajet optique du faisceau de second harmonique et un second moyen de changement d'angle d'incidence (16, 22) destiné à changer un angle d'incidence du faisceau de second harmonique par rapport à l'élément optique (15, 70), et
le moyen de commande (40) contrôle le premier moyen de changement (14, 20) et le second moyen de changement (16, 22) sur la base du changement de puissance du faisceau fondamental.

3. Appareil laser médical selon la revendication 2, dans lequel l'élément optique (15) possède un indice de réfraction et une longueur identiques à l'élément de conversion de longueur d'onde (13), et
le moyen de commande (40) contrôle le premier moyen de changement (14, 20) et le second moyen de changement (16, 22) afin de changer l'angle d'incidence du faisceau fondamental par rapport à l'élément de conversion de longueur d'onde et l'angle d'incidence du faisceau de second harmonique par rapport à l'élément optique afin que les deux angles soient égaux, mais dans des directions opposées.

4. Appareil laser médical selon l'une quelconque des revendications 1 à 3, comprenant en outre :
un moyen de régulation de la température (21, 23) destiné à changer une température de l'élément de conversion de longueur d'onde (13) ; et
un détecteur de puissance (31) qui détecte la puissance du faisceau de second harmonique ;
et
dans lequel le moyen de commande contrôle le moyen de régulation de la température (21, 23) sur la base d'un résultat de détection du détecteur de puissance.
